(19) Europäisches Patentamt
European Patent Office
Office européen des brevets

(11) **EP 2 199 277 B1**

(12) **EUROPÄISCHE PATENTSCHRIFT**

(45) Veröffentlichungstag und Bekanntmachung des Hinweises auf die Patenterteilung:
**28.09.2011 Patentblatt 2011/39**

(51) Int Cl.:
*C07C 263/10* (2006.01)   *C07C 265/00* (2006.01)
*C07C 265/14* (2006.01)   *B01J 19/26* (2006.01)
*B01J 19/18* (2006.01)

(21) Anmeldenummer: **09015171.3**

(22) Anmeldetag: **08.12.2009**

(54) **Verfahren zur Herstellung von Isocyanaten in der Gasphase**

Method for manufacturing isocyanates in the gaseous phase

Procédé destiné à la fabrication d'isocyanates en phase gazeuse

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **18.12.2008 DE 102008063728**

(43) Veröffentlichungstag der Anmeldung:
**23.06.2010 Patentblatt 2010/25**

(73) Patentinhaber: **Bayer MaterialScience AG**
**51368 Leverkusen (DE)**

(72) Erfinder:
• **Bruns, Rainer, Dr.**
**51373 Leverkusen (DE)**
• **Pohl, Fritz, Dr.**
**25541 Brunsbüttel (DE)**
• **Steffens, Friedhelm**
**51373 Leverkusen (DE)**
• **Michele, Volker, Dr.**
**51065 Köln (DE)**

(56) Entgegenhaltungen:
**EP-A1- 1 449 826    EP-A2- 0 291 819**
**EP-A2- 1 319 655    GB-A- 1 165 831**

## Beschreibung

Die Erfindung betrifft ein Verfahren zur Herstellung von primären Isocyanaten durch Umsetzung der entsprechenden primären Amine mit Phosgen in der Gasphase.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen. Eine Möglichkeit der Herstellung von Isocyanaten ist die Umsetzung der Amine mit dem Phosgen in der Gasphase. Es sind aus dem Stand der Technik verschiedene Verfahren zur Herstellung von Isocyanaten durch Umsetzung von Aminen mit Phosgen in der Gasphase bekannt.

GB-A-1 165 831 beschreibt ein Verfahren zur Herstellung von Isocyanaten in der Gasphase, bei dem die Umsetzung des dampfförmigen Amins mit dem Phosgen bei Temperaturen zwischen 150°C und 300°C in einem mit einem mechanischen Rührer ausgestatteten und über einen Heizmantel temperierbaren Rohrreaktor durchgeführt wird. Der in GB 1 165 831 offenbarte Reaktor ähnelt einem Dünnschichtverdampfer, dessen Rührer die in den Reaktionsraum eintretenden sowie die in dem Reaktionsraum vorhandenen Gase mischt und gleichzeitig die mit dem Heizmantel umgebenen Wände des Rohrreaktors bestreicht, um so einen Aufbau von polymeren Material an der Rohrwand zu verhindern, da ein solcher Aufbau den Wärmeübergang erschweren würde. Nach der Lehre von GB 1 165 831 wird die Mischung der in den Reaktionsraum eintretenden Eduktströme ausschließlich durch den mit etwa 1000 Umdrehungen pro Minute laufenden Rührer erreicht, dessen Antrieb extern über eine die Reaktorwandung durchdringende Welle erfolgt. Nachteilig an dem offenbarten Verfahren ist zum einen die Verwendung eines schnell laufenden Rührers und dessen externer Antrieb über eine die Reaktorwandung durchdringende Welle, da ein solches Rührwerk beim Einsatz von Phosgen einen sehr hohen Aufwand zur sicherheitstechnisch notwendigen Abdichtung des Reaktors erfordert. Weiterhin nachteilig ist, dass die Vermischung der Gase nur durch den Rührer erreicht wird, was trotz der angewandten hohen Drehzahlen zu langen Mischzeiten und in folge davon zu einer breiten Kontaktzeitverteilung der Reaktanden führt, die nach der Lehre von EP-A-570 799 wiederum zu unerwünschter Feststoffbildung führt.

EP-A-289 840 beschreibt die Herstellung von (cyclo)aliphatischen Diisocyanaten durch Gasphasenphosgenierung, wobei EP-A-289 840 die Umsetzung der Amine mit dem Phosgen in einem zylindrischen Raum ohne bewegte Teile in einer turbulenten Strömung bei Temperaturen zwischen 200°C und 600°C sowie Reaktionszeiten in der Größenordnung von $10^{-4}$ Sekunden offenbart. Durch den Verzicht auf bewegte Teile, die mit die Reaktorwandung durchdringenden Antrieben ausgerüstet sind, wird zum einen die Gefahr eines Phosgenaustrittes reduziert. Nach der Lehre von EP-A-289 840 werden die Gasströme an einem Ende des Rohrreaktors in diesen durch eine Düse und einen Ringspalt zwischen Düse und Mischrohr in den Reaktor eingebracht und dadurch vermischt. Somit offenbart diese Schrift zum anderen eine Weiterentwicklung der Mischtechnologie, indem die Vermischung der Gase vorteilhaft durch ein statisches Mischorgan, nämlich Düse und Ringspalt, anstelle des in GB 1 165 831 offenbarten Rührers erfolgt. Gemäß der Lehre der EP-A-289 840 ist es für die Durchführbarkeit des in der EP-A-289 840 offenbarten Verfahrens wesentlich, dass die Abmessungen des Rohrreaktors und die Strömungsgeschwindigkeiten im Reaktionsraum so bemessen werden, dass in dem Reaktionsraum eine turbulente Strömung herrscht, die gemäß der Lehre von EP-A-289 840 durch eine Reynoldszahl von mindestens 2500, vorzugsweise mindestens 4700 charakterisiert wird. Nach der Lehre von EP-A-289 840 ist diese Turbulenz im Allgemeinen dann gewährleistet, wenn die gasförmigen Reaktionspartner den Reaktionsraum mit einer Strömungsgeschwindigkeit von mehr als 90 m/s durchlaufen. Durch die turbulente Strömung im zylindrischen Raum (Rohr) wird, wenn man von Fluidelementen in Wandnähe absieht, eine relative gute Strömungsgleichverteilung im Rohr und damit eine relativ enge Verweilzeitverteilung erreicht, die, wie in EP-A-570 799 beschrieben, zu einer Verringerung der Feststoffbildung führen. Nachteilig an dem in EP-A-289 840 offenbarten Verfahren ist, dass wegen der notwendigen hohen Strömungsgeschwindigkeiten die Realisierung der zur vollständigen Umsetzung der Amine notwendigen Verweilzeit, speziell bei Einsatz aromatischer Amine, nur in sehr langen Misch- und Reaktorrohren möglich ist.

EP-A-570 799 betrifft ein Verfahren zur Herstellung von aromatischen Diisocyanaten, dadurch gekennzeichnet, dass die Umsetzung des zugehörigen Diamins mit dem Phosgen in einem Rohrreaktor oberhalb der Siedetemperatur des Diamins innerhalb einer mittleren Kontaktzeit der Reaktanden von 0,5 bis 5 Sekunden durchgeführt wird. Wie in der Schrift beschrieben ist, führen sowohl zu lange als auch zu kurze Reaktionszeiten zu einer unerwünschten Feststoffbildung. Es wird daher ein Verfahren offenbart, bei dem die mittlere Abweichung von der mittleren Kontaktzeit weniger als 6% beträgt.

Die Einhaltung dieser Kontaktzeitverteilung wird zum einen dadurch erreicht, dass die Reaktion in einer Rohrströmung durchgeführt wird, die entweder durch eine Reynolds-Zahl von oberhalb 4.000 oder einer Bodenstein-Zahl von oberhalb 100 charakterisiert wird. Nach der Lehre von EP-A-570 799 wird dadurch eine zu 90% angenäherte Pfropfenströmung erreicht und weisen alle Volumenteile der Strömung weitgehend die gleichen Strömungszeiten auf, so dass durch die annähernd gleichen Verweilzeiten aller Volumenteile eine möglichst geringe Verbreiterung der Verteilung der Kontaktzeit zwischen den Reaktionspartnern erfolgt.

Nach der Lehre von EP-A-570 799 wird zum anderen die Abweichung von der mittleren Kontaktzeit bei der praktischen Durchführung des Verfahrens jedoch auch wesentlich durch die notwendige Zeit zur Vermischung der Reaktionspartner bestimmt. EP-A-570 799 führt aus, dass solange die Reaktionspartner noch nicht homogen vermischt sind, im Reaktionsraum noch Gasvolumina vorhanden sind, die noch nicht mit dem Reaktionspartner in Kontakt treten konnten und somit abhängig von der Durchmischung bei gleichen Strömungszeiten der Volumenteile unterschiedliche Kontaktzeiten der Reaktionspartner erhalten werden. Nach der Lehre von EP-A-570 799 soll daher die Vermischung der Reaktionspartner innerhalb einer Zeit von 0,1 bis 0,3 s bis zu einem Segregationsgrad von $10^{-3}$ erfolgen, wobei der Segregationsgrad als Maß für die Unvollständigkeit der Durchmischung dient (siehe z.B. Chem.-Ing.-Techn. 44 (1972), S.1051 ff; Appl.Sci.Res.(the Hague) A3 (1953),p.279). EP-A-570 799 offenbart, dass zur Erzeugung entsprechend kurzer Mischzeiten im Prinzip bekannte Methoden auf Basis von Mischaggregaten mit bewegten oder statischen Mischorganen, bevorzugt statischen Mischorganen, eingesetzt werden können, wobei nach der Lehre von EP-A-570 799 insbesondere die Anwendung des Strahlmischerprinzips (Chemie-Ing.-Techn. 44 (1972) S.1055, Abb.10) ausreichend kurze Mischzeiten liefert.

Beim Strahlmischerprinzip (Chemie-Ing.-Techn. 44 (1972) S. 1055, Abb. 10) werden dem Rohrreaktor zwei Eduktströme I und II zugeführt, wobei der Eduktstrom I über eine Zentraldüse und der Eduktstrom II über einen Ringraum zwischen Zentraldüse und Rohrreaktorwand zugeführt werden. Die Strömungsgeschwindigkeit des Eduktstroms I ist dabei groß gegenüber der Strömungsgeschwin-digkeit des Eduktstroms II. Nach einer vom Düsendurchmesser und vom Unterschied den Strömungsgeschwindigkeiten der Edukte abhängigen Zeit bzw. nach dem entsprechenden Weg wird dann die vollständige Vermischung der Edukte erreicht.

Nachteilig an dem Strahlmischerprinzip ist, dass bei Vergrößerung der Reaktoren, die häufig als Rohrreaktoren ausgebildet sind, auch eine Vergrößerung der Mischdüse, die häufig als Glattstrahldüse ausgebildet ist, notwendig wird. Mit der Vergrößerung des Durchmessers der Glattstrahldüse wird aber auch die Geschwindigkeit der Vermischung des Zentralstrahls durch den größeren erforderlichen Diffusionsweg verringert, somit die Mischzeit entsprechend verlängert. Zudem wird die Gefahr von Rückvermischung erhöht, was im Fall der Umsetzung von primären Aminen mit Phosgen in der Gasphase wie oben ausgeführt wiederum zur Entstehung polymerer Verunreinigungen und damit fester Anbak-kungen im Reaktor führt. Somit ist bei der Übertragung der Gasphasenphosgenierung primärer Amine in ein großtechnisch genutztes Verfahren ein einfaches Übertragen der Geometrie auf großtechnisch sinnvolle Größenordnungen nicht möglich, da der Durchmesser des Innenrohres so erhöht werden müsste, dass eine Durchmischung der Edukte aufgrund der langen quer zur Strömungsrichtung stehenden Strecken in den erforderlichen kurzen Mischzeiten ohne Zusatzmaßnahmen nicht mehr möglich ist.

Die Optimierung des **Einsatzes von Rohrreaktoren** für die Gasphasenphosgenierung, wie er grundlegend in der EP-A-570 799 unter Anwendung des Strahlmischerprinzips (Chemie-Ing.-Techn. 44 (1972) S.1055, Abb.10) offenbart wurde, ist deshalb Gegenstand zahlreicher Anmeldungen, die auf eine Verbesserung der Mischung der Eduktströme durch eine Weiterentwicklung des statischen Mischorgans abzielen.

Gemäß der Lehre von EP-A-1 526 129 hat eine **Erhöhung der Turbulenz** des Eduktstromes in der Zentraldüse einen positiven Einfluss auf die Durchmischung der Reaktanden und damit auf die Gasphasenreaktion insgesamt. Als Folge der besseren Durchmischung nimmt die Neigung zur Nebenproduktbildung ab und die erforderliche Verweilzeit und somit Reaktorbaulänge sinken deutlich. EP-A-1 526 129 offenbart bei einem Einsatz einer Spiralwendel als Turbulenz erzeugendem Einbauelement in der Zentraldüse eine Verkürzung der Mischstrecke auf 42% der ursprünglichen Länge.

Nach der Lehre von EP-A-1 555 258 können die Nachteile, die sich bei der Gasphasenphosgenierung primärer Amine aus der Vergrößerung der Reaktoren und der damit einhergehenden Vergrößerung der Mischdüse mit der Folge der Verlängerung der Mischzeiten entstehen, eliminiert werden, wenn der eine Eduktstrom über einen Ringspalt, der konzentrisch im Strom des anderen Eduktes angebracht ist, mit hoher Geschwindigkeit eingedüst wird. Nach der Lehre von EP-A-1 555 258 werden dadurch die Diffusionswege für die Vermischung klein und die Mischzeiten sehr kurz. Gemäß der Lehre von EP-A-1 555 258 kann so die Umsetzung der primären Amine mit Phosgen in der Gasphase mit hoher Selektivität zum gewünschten Isocyanat durchgeführt und somit die Entstehung von polymeren Verunreinigungen und die Ausbildung von Anbackungen deutlich verringert werden. Wie in der EP-A-1 555 258 weiterhin offenbart, sind - bei vergleichbaren Geschwindigkeiten der Komponenten an der Mischstelle - bei dem erfindungsgemäßen Verfahren deutlich kürzere Reaktionsräume zur Erzielung der Maximaltemperatur im Reaktionssystem als bei dem Einsatz konventioneller Glattstrahldüsen erforderlich. Entsprechend kann gegenüber dem Stand der Technik die Umsetzung der primären Amine mit Phosgen zu den entsprechenden Isocyanaten in deutlich kürzeren Reaktoren erfolgen. Nachteilig an dem offenbarten Verfahren ist, dass sowohl der Zentralstrom sehr gleichmäßig über den konzentrischen Ringspalt als auch der zweite Eduktstrom sehr gleichmäßig in den äußeren und inneren Ringraum verteilt werden muss, da es andernfalls zu einer instabilen Reaktionsführung in dem Reaktionsraum, gemäß der Lehre von EP-A-1 362 847 erkennbar an Temperaturschwankungen und Asymmetrien in der Temperaturverteilung im Reaktionsraum, kommt. Die erforderliche sehr gleichmäßige Verteilung beider Eduktströme ist konstruktiv aufwendig, zudem können bereits kleinste Feststoffmengen, deren Entstehung bei der Synthese der Isocyanate im technischen Maßstab nicht vollständig auszuschließen ist, zum Verstopfen des Ringspaltes führen und damit die Verfügbarkeit der Isocyanatanlage herabsetzen.

Nach der Lehre von EP-A-1 449 826 können die sich aus der Vergrößerung der Reaktoren und der damit einhergehenden Vergrößerung der Mischdüse ergebenen Nachteile durch die Aufteilung des Zentralstromes auf mehrere Düsen umgangen werden. EP-A- 1 449 826 offenbart ein Verfahren zur Herstellung von Diisocyanaten durch Phosgenierung der entsprechenden Diamine, bei dem die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und in einem Rohrreaktor vermischt und zur Reaktion gebracht werden, dadurch gekennzeichnet, dass in dem Rohrreaktor eine Anzahl n ≥2 von parallel zur Achse des Rohrreaktors ausgerichtete Düsen angeordnet sind, wobei der die Diamine enthaltende Strom dem Rohrreaktor über die n Düsen zugeführt wird und der Phosgenstrom dem Rohrreaktor über den verbleibenden freien Raum zugeführt wird. Nach der Lehre der EP-A-1 449 826 wird durch das erfindungsgemäße Verfahrens eine Verkürzung der Mischzeiten gegenüber einer Einfachdüse (einzelnen Düse) mit gleicher Querschnittsfläche erreicht. Durch die wesentlich kürzeren Mischzeiten wird - wie oben ausgeführt - zum einen die Verteilung der Kontaktzeit der Reaktanden positiv beeinflusst. Zum anderen machen die wesentlich kürzeren Mischzeiten bei gleicher Kontaktzeit der Reaktanden deutlich geringere Verweilzeiten im Reaktionsraum erforderlich und lassen so den Einsatz von Reaktionsräumen deutlich geringerer Länge zu.

Bei der Übertragung des in EP-A-1 449 826 offenbarten Verfahrens in eine großtechnische Dimension können die erforderlich kurzen Mischzeiten nach dem Stand der Technik nur durch entsprechend erhöhte Eintrittsgeschwindigkeiten der Reaktanden in den Reaktionsraum erzielt werden, wie es gemäß der Lehre von WO2008/055898 auch bei Einsatz alternativer Düsenkonfigurationen als Mischorgan erforderlich ist. Nachteilig an erhöhten Eintrittsgeschwindigkeiten ist, dass die hohen Strömungsgeschwindigkeiten bei der Realisierung der zur vollständigen Umsetzung der Amine notwendigen Verweilzeit, speziell bei Einsatz aromatischer Amine, nur in sehr langen Misch- und Reaktorrohren möglich ist.

EP-A-1 319 655 offenbart ein Herstellungsverfahren für (Poly-)Isocyanate in der Gasphase mit optimierter Vermischung der Reaktanden Diamin und Phosgen. Zu diesem Zweck wird ein Verfahren offenbart, in welchem die dampfförmigen Diamine und Phosgen durch ein statisches Mischorgan mit genau spezifizierter Geometrie, wobei die beiden gasförmigen Edukte parallel einen Reaktor des Durchmessers D durchströmen, wobei ein Edukt E1 über eine Düse mit dem Öffnungsdurchmesser d zentral in einen Strom des Eduktes E2 eingebracht wird, wobei die Gasgeschwindigkeit von E1 groß ist gegenüber der Gasgeschwindigkeit von E2, und wobei das Amin durch die Düse fließt, während Phosgen durch den Ringraum um die Düse zugeführt wird, und wobei das Verhältnis von engstem Düsendurchmesser d zu Reaktordurchmesser D im Bereich von 5 % bis 45 % liegt, gemischt und kontinuierlich in einem Reaktionsraum ohne sich bewegende Teile zur Reaktion gebracht werden. Der Reaktionsraum enthält gemäß EP-A-1 319 655 ausdrücklich keine sich bewegenden Teile, d. h. eine Kombination aus Düse und beweglichem Mischorgan wird nicht offenbart.

EP-A-0 291 819 offenbart ein Verfahren zum Herstellen von Isocyanaten durch

a) Vermischen und Umsetzen von Lösungen bzw. Suspensionen primärer Amine bzw. deren Salze mit Phosgenlösung, wobei die beiden Stoffe zum Einleiten der Umsetzung kontinuierlich in eine Mischzone, welche mindestens eine Rotorscheibe aufweist, eingebracht werden und das entstandene Vorprodukt wieder ausgeführt wird,

b) weitere Umsetzung der entstandenen Primärprodukte unter Erhitzen,

c) Aufarbeitung der erhaltenen Isocyanatlösung zu den entsprechenden Roh- und/oder Reinisocyanaten,

wobei bei der Durchführung des Schrittes a) die Phosgenlösung axial zur Rotorscheibe zugefügt wird und das in Lösung oder Suspension befindliche Amin parallel zum aber mit Abstand vom Strom der Phosgenlösung gegen die Rotorscheibe verdüst wird. Ein Gasphasenverfahren wird in EP-A-0 291 819 nicht offenbart.

Aufgabe der vorliegenden Erfindung war es daher, ein Verfahren zur Herstellung von Isocyanaten durch Umsetzung von primären Aminen mit Phosgen in der Gasphase im großtechnischen Maßstab zu ermöglichen, das bei gleicher Eintrittsgeschwindigkeit der Reaktanden eine schnellere Durchmischung der Reaktanden bei gleichzeitig geringer Verstopfungsgefahr der Mischaggregate beinhaltet.

Diese Aufgabe konnte überraschend dadurch gelöst werden, dass das primäre Amin und das Phosgen in einem Rohrreaktor oberhalb der Siedetemperatur des Amins in der Gasphase umgesetzt werden, wobei die Eduktströme über eine Düsenanordnung in den Reaktionsraum eintreten, wobei ein Eduktstrom über n ≥1 parallel zur Achse des Reaktionsraumes ausgerichteter Düsen und der zweite Eduktstrom über den die Düsen umgebenden freien Raum dem Reaktionsraum zugeführt wird und wobei der Reaktionsraum zumindest ein bewegliches Mischorgan enthält. Überraschenderweise kann durch die Kombination des Strahlmischerprinzips mit einem beweglichen Mischorgan die Vergrößerung der Reaktoren und der Mischdüse / Mischdüsen in einen großtechnischen Maßstab auch ohne eine Erhöhung der Eintrittsgeschwindigkeit der Reaktanden oder den Einsatz verstopfungsanfälliger Düsenkonfigurationen mit dennoch ausreichend kurzen Mischzeiten der Eduktströme erreicht werden. Die vorteilhafte Kombination eines statischen mit einem beweglichen Mischorgan war für den Fachmann nicht vorherzusehen, da nach dem Stand der Technik sich der Einsatz beweglicher Mischorgane als nicht vorteilhaft für schnelle Gasphasenreaktionen herausgestellt hat.

Gegenstand der Erfindung ist daher ein Verfahren zur Herstellung primärer Isocyanate durch Umsetzung der entsprechenden primären Amine mit Phosgen, dadurch gekennzeichnet, dass die primären Amine oberhalb der Siedetemperatur der Amine mit Phosgen in einem Rohrreaktor, der einen Reaktionsraum enthält, umgesetzt werden, bei dem

a) mindestens ein Phosgen enthaltender Eduktstrom P und mindestens ein die Amine enthaltender Eduktstrom A dem Reaktionsraum über eine Düsenanordnung zugeführt werden, wobei die Düsenanordnung eine Anzahl von n ≥1 parallel zur Rotationsachse des Rohrreaktors ausgerichteter Düsen und einen die Düsen umgebenden freien Raum umfasst, und

b) einer der Eduktströme A oder P über die Düsen und der andere Eduktstrom über den die Düsen umgebenden freien Raum dem Reaktionsraum zugeführt werden, und

c) der Reaktionsraum zumindest ein bewegliches Mischorgan enthält.

Üblicherweise enthält der Rohrreaktor einen zur Strömungsrichtung im Wesentlichen rotationssymmetrischen Reaktionsraum. "Rotationssymmetrisch" bedeutet dabei in Übereinstimmung mit dem Stand der Technik, siehe z.B. WO 2007/028 715 A1, S. 3, Z. 28 ff, dass ein Körper oder Raum, hier der Reaktionsraum, bei Drehung um die Rotationsachse eine Drehsymmetrie aufweist. Dabei kann es sich beispielsweise um eine zweizählige Drehachse (C2), um eine dreizählige (C3) oder vierzählige Drehachse (C4) handeln oder bevorzugt um eine vollständige Drehsymmetrie (C∞). So besitzt beispielsweise eine von einer Ellipse berandete Fläche eine zweizählige Drehachse. Als weiteres Beispiel besitzt eine von einem Kreis berandete Fläche eine vollständige Drehsymmetrie.

Besonders bevorzugt handelt es sich dabei um einen Rohrreaktor mit einer sich, ggf. auch nur abschnittsweise, in Strömungsrichtung erweiternden, gleichbleibenden und/oder abnehmenden durchströmten Querschnittsfläche.

Nicht bevorzugt, aber prinzipiell auch möglich sind Reaktionsräume, die ovale oder aus beliebigen geschlossenen ebenen Polygonen zusammengesetzte Strömungsquerschnitte aufweisen.

Unter parallel zur Achse des Rohrreaktors ausgerichteten Düsen im Sinne dieser Erfindung soll verstanden werden, dass die Winkel-Abweichung zwischen der Ausrichtung der Achsenmitte der jeweiligen Düsen und der Ausrichtung der Achsenmitte des Reaktors weniger als 5 Grad, bevorzugt weniger als 3,5 Grad beträgt.

In einer bevorzugten Ausführungsform der vorliegenden Erfindung haben die n parallel zur Achse des Rohrreaktors ausgerichteten Düsen, sofern n eine ganze positive Zahl von größer 1 ist, bevorzugt den gleichen Durchmesser, insbesondere bevorzugt sind die Einzeldüsen im Rahmen der Fertigungstoleranzen baugleich.

Die Anordnung der n parallel zur Achse des Rohrreaktors ausgerichtete Düsen, sofern n eine ganze positive Zahl von größer 1 ist, erfolgt bevorzugt auf einem Kreisring um die Reaktorachse. Sofern n > 2 Einzeldüsen eingesetzt werden, können in einer weiteren Ausführungsform n-1 Einzeldüsen auf einem Kreisring um eine zentral angeordnete Düse angebracht werden. Insbesondere erfolgt die Anordnung der n parallel zur Achse des Rohrreaktors ausgerichtete Düsen rotationssymmetrisch, wobei n eine ganze positive Zahl von größer 1 ist.

In einer weiteren bevorzugten Ausführungsform der vorliegenden Erfindung werden die n parallel zur Achse des Rohrreaktors ausgerichteten Düsen, wobei n eine ganze positive Zahl von mindestens 1 ist, über jeweils ein flexibles oder starres Verbindungsstück mit einem Einlass für einen der Eduktströme verbunden. Starre Verbindungsstücke können Rohrleitungsstücke sein, flexible Verbindungsstücke können z.B. Schläuche oder bevorzugt Kompensatoren sein.

In einer bevorzugten Ausführungsform des erfindungsgemäßen Verfahrens wird das Amin (d.h. der mindestens eine die Amine enthaltende Eduktstrom) über die n parallel zur Achse des Rohrreaktors ausgerichteten Düsen dem Reaktor zugeführt, wobei n eine ganze positive Zahl von mindestens 1 ist. Das Phosgen (d.h. der mindestens eine das Phosgen enthaltende Eduktstrom) wird in dieser Ausführungsform in den die Düsen umgebenden Raum eingeführt, d.h. in den Raum, der von der Reaktorwand und der zumindest einen Amindüse begrenzt wird. Sofern der Aminstrom über nur eine Düse dem Reaktionsraum zugeführt wird, d.h. n = 1 ist, ist diese Düse bevorzugt zentral auf der Längsachse des Reaktionsraums im Reaktor positioniert.

In einer alternativen Ausführungsform des erfindungsgemäßen Verfahrens wird das Phosgen (d.h. der mindestens eine das Phosgen enthaltende Eduktstrom) über die n parallel zur Achse des Rohrreaktors ausgerichteten Düsen dem Reaktor zugeführt, wobei n eine ganze positive Zahl von mindestens 1 ist. Das Amin (d.h. der mindestens eine die Amine enthaltende Eduktstrom) wird in dieser Ausführungsform in den die Düsen umgebenden Raum eingeführt, d.h. in den Raum, der von der Reaktorwand und der zumindest einen Phosgendüse begrenzt wird. Sofern der Phosgenstrom über nur eine Düse dem Reaktor zugeführt wird, d.h. n = 1 ist, ist diese Düse bevorzugt zentral auf der Längsachse des Reaktionsraums im Reaktor positioniert.

Bevorzugt treten bei dem erfindungsgemäßen Verfahren die bevorzugt kontinuierlich eingespeisten Eduktströme mit einem Geschwindigkeitsverhältnis von 2 - 20, besonders bevorzugt von 3 - 15, ganz besonders bevorzugt von 4 - 12, in den Reaktionsraum ein. Bevorzugt tritt der Eduktstrom, der über die n parallel zur Achse des Rohrreaktors ausgerichteten Düsen dem Reaktionsraum zugeführt wird, mit der höheren Strömungsgeschwindigkeit in den Reaktionsraum ein. Insbesondere bevorzugt ist dieser Eduktstrom der Amin-haltige Eduktstrom A.

In einer besonderen Ausführungsform des erfinderischen Verfahrens können die n ≥1 parallel zur Achse des Rohrreaktors ausgerichteten Düsen bzw. für n = 1 die bevorzugt zentral im Reaktor positionierte Düse mit zusätzlichen Turbulenz erzeugenden Elementen, wie z.B. Wendeln, Spiralwendeln oder schräg in die Strömung eingebrachten runde oder eckige Platten ausgerüstet sein.

In einer weiteren bevorzugten Ausführungsform des erfindungsgemäßen Verfahrens befindet sich in dem die Düsen umgebenden freien Raum, der durch die Reaktorwand und die n ≥1 Düsen begrenzt wird, zumindest ein, bevorzugt mindestens zwei Strömungsvergleichmäßiger und / oder Strömungsgleichrichter, die die Geschwindigkeit der Strömung in diesem Raum über den gesamten Querschnitt dieses Raumes vergleichmäßigen. Beispielsweise können als Strömungsvergleichmäßiger Lochbleche, Siebe, Sintermetall, Fritten oder Schüttungen, bevorzugt Lochbleche, eingesetzt werden. Der Einsatz von z.B. Wabenstrukturen und Rohrstrukturen als Strömungsgleichrichter, wie in EP-A-1 362 847 offenbart, ist ebenfalls möglich.

Im Gegensatz zu einem statischen Mischorgan ist unter einem beweglichen Mischorgan im Sinne der vorliegenden Erfindung ein sich drehendes oder oszillierend bewegendes Element zu verstehen. Geeignete Mischorgane sind beispielsweise Rührer wie Propellerrührer, Schrägblattrührer, Scheibenrührer, Impellerrührer, Kreuzbalkenrührer, Ankerrührer, Blatt- oder Gitterrührer, Wendelrührer oder Zahnscheibenrührer. Der Rührer kann einen oder mehrere Flügel, Blätter, Scheiben, Arme oder Anker besitzen, die auf einer Welle montiert sind, bevorzugt werden Flügel oder Blätter. Die Flügel, Blätter, Scheiben, Arme oder Anker können auf unterschiedlichen Positionen entlang der Welle montiert sein, bevorzugt sind sie auf der gleichen Position entlang der Welle montiert. Ganz besonders bevorzugt sind sie am Ende der Welle montiert. Bevorzugt hat das bewegliche Mischorgan mehr als einen Flügel bzw. mehr als ein Blatt. Die Flügel oder Blätter können schräg angestellt oder gerade sein, weiterhin können sie beliebig geformt oder gebogen sein.

Die Drehgeschwindigkeit des beweglichen Mischorgans kann langsam oder schnell sein, wobei schnell definiert wird mit > 1000 Umdrehungen pro Minute (UpM) und langsam definiert wird mit ≤1000 Umdrehungen pro Minute. Bevorzugt hat das bewegte Mischorgan eine langsame Drehgeschwindigkeit.

Der Antrieb des zumindest einen beweglichen Mischorgans kann mit verschiedenen Methoden erfolgen. Insbesondere kann der Antrieb durch ein externes Antriebsorgan oder unter Nutzung des Impulses mindestens eines der dem Reaktionsraum zugeführten Eduktströme erfolgen. Insbesondere bevorzugt erfolgt der Antrieb des zumindest einen beweglichen Mischorgans so, dass die beweglichen Ausrüstungsteile des jeweiligen Mischorgans, beispielsweise die Welle, nicht durch die Reaktorwand geführt werden, wodurch die sicherheitstechnisch schwierige Abdichtung der Welle beim Einsatz von heißem Phosgengas vermieden wird.

Unter externen Antriebsorganen im Sinne dieser Erfindung sind solche Antriebsorgane zu verstehen, die sich außerhalb des Reaktors befinden. Als geeignete externe Antriebsorgane seien hier beispielsweise Motoren, insbesondere elektrische Motoren, genannt, wobei die Übertragung der Antriebsenergie bevorzugt indirekt, d.h. ohne dass ein bewegliches Element des zumindest einen beweglichen Mischorgans durch die Reaktorwand geführt wird, auf das bewegliche Mischorgan übertragen wird. Als geeignete indirekte Antriebsmittel seien hier beispielsweise magnetgekuppelte Antriebe genannt.

Weiterhin kann der Impuls zumindest eines der Eduktströme zum Antrieb des zumindest einen beweglichen Mischorgans verwendet werden. Dazu kann einerseits der Impuls der in den Reaktionsraum durch die n ≥1 von parallel zur Rotationsachse des Rohrreaktors ausgerichteten Düsen und / oder durch den die Düsen umgebenden freien Raum eingetretenen Eduktströme A und / oder P zum Antrieb des beweglichen Mischorgans verwendet werden, d.h. in diesem Fall wird der Impuls der Strömung im Reaktionsraum zum Antrieb des beweglichen Mischorgans im Reaktionsraum verwendet. Weiterhin kann andererseits das zumindest eine bewegliche Mischorgan im Reaktionsraum bevorzugt über zumindest eine Welle mit einem Antriebspropeller verbunden sein, wobei sich der Antriebspropeller außerhalb des Reaktionsraumes befindet. Bevorzugt befindet sich der Antriebspropeller in Strömungsrichtung vor Eintritt in den Reaktionsraum, und zwar in dem Eduktstrom A und oder dem Eduktstrom P. In einer besonderes bevorzugten Ausführungsform befindet sich der Antriebspropeller in dem über die n ≥1 parallel zur Rotationsachse des Rohrreaktors ausgerichteten Düsen zugeführten Eduktstrom. In weiterer ebenso besonders bevorzugter Ausführungsform befindet sich der Antriebspropeller in dem Eduktstrom, der durch den Bereich des die Düsen umgebenden freien Raums zugeführt wird.

Der Antriebspropeller kann ein oder mehrere Flügel, Blätter, Scheiben, Arme oder Anker besitzen, bevorzugt werden Flügel oder Blätter. Bevorzugt besitzt der Antriebspropeller mehr als einen Flügel bzw. mehr als ein Blatt, bevorzugt sind diese schräggestellt auf der Welle montiert.

Sofern mehr als ein bewegliches Mischorgan eingesetzt wird, erfolgt der Antrieb der mehreren beweglichen Mischorgane bevorzugt nach derselben Methode, d.h. bevorzugt werden alle beweglichen Mischorgane durch ein externes Antriebsorgan oder unter Nutzung des Impuls zumindest eines der Eduktströme angetrieben. Bevorzugt hat jedes bewegliche Mischorgan einen separaten Antriebspropeller, es ist aber auch denkbar, dass ein Antriebspropeller mehrere bewegliche Mischorgane antreibt. Bevorzugt treibt ein externes Antriebsorgan nur ein bewegliches Mischorgan an, es ist aber auch denkbar, dass ein externes Antriebsorgan mehrere bewegliche Mischorgane antreibt. Bevorzugt ist jedes bewegliche Mischorgan mit einem Antriebspropeller verbunden, es ist aber auch denkbar, dass jedes bewegliche Mischorgan von mehr als einem Antriebspropeller angetrieben wird.

Das bewegliche Mischorgan befindet sich in dem Reaktionsraum. Im Sinne der vorliegenden Erfindung beginnt der Reaktionsraum mit Austritt der Strömung in Strömungsrichtung aus den n ≥1 parallel zur Achse des Rohrreaktors ausgerichteten Düsen, wobei n eine ganze positive Zahl von mindestens 1 ist. Mit Austritt der Strömung aus den n ≥1 parallel zur Achse des Rohrreaktors ausgerichteten Düsen beginnt die Vermischung der Eduktströme, die - wie oben ausgeführt - aufgrund der Schnelligkeit der Reaktionen bei der Gasphasenphosgenierung von primären Aminen sofort durch die einsetzende Umsetzung der Gase überlagert wird.

Das zumindest eine bewegliche Mischorgan kann in der Reaktionszone an jeder beliebigen Position angebracht sein. Bevorzugt befindet sich das zumindest eine bewegliche Mischorgan weniger als 5xD in Strömungsrichtung entfernt von dem Anfang des Reaktionsraumes, insbesondere bevorzugt weniger als 3xD. Dabei wird unter D der größte Durchmesser des Reaktionsraums auf Höhe der Austrittsöffnung aus der Düse verstanden. Sofern mehrere bewegliche Mischorgane in der Reaktionszone vorhanden sind, haben diese bevorzugt die gleiche Position in der Reaktionszone.

Das bewegliche Mischorgan kann sich zentral auf der Achse des Reaktors befinden, es ist aber auch eine exzentrische Anbringung des beweglichen Mischorgans bzgl. der Achse des Reaktors denkbar.

Sofern mehr als ein bewegliches Mischorgan in dem Reaktionsraum eingesetzt wird, sind diese bevorzugt auf einem Kreisring um die Reaktorachse angebracht. In einer weiteren Ausführungsform können die beweglichen Mischorgane auf einem Kreisring um ein zentral angeordnetes bewegliches Mischorgan angebracht werden. Sofern mehr als ein bewegliches Mischorgan eingesetzt wird, erfolgt deren Anordnung bevorzugt symmetrisch.

In einer weiteren bevorzugten Ausführungsform befinden sich im Reaktor n > 1 parallel zur Achse Reaktionsraumes ausgerichtete Düsen und m ≥1 bewegliche Mischorgane, wobei n und m jeweils ganze positive Zahlen sind, wobei die gesamte Anordnung bevorzugt symmetrisch erfolgt. In einer weiteren besonders bevorzugten Ausführungsform ist die Anordnung der n > 1 parallel zur Achse des Reaktionsraumes ausgerichtete Düsen und m ≥ 1 bewegliche Mischorgane, wobei n und m jeweils ganze positive Zahlen sind, bevorzugt symmetrisch in Bezug auf die Reaktorachse.

Die Flügel, Blätter, Scheiben, Arme oder Anker des zumindest einen beweglichen Mischorgans können verschiedenen Längen haben. Die maximale Länge wird bei Verwendung eines durch eine vollständige Drehsymmetrie gekennzeichneten Reaktionsraumes durch den halben Durchmesser des Reaktors begrenzt, wird im Gegensatz dazu ein durch eine C2-Symmetrie gekennzeichneter Reaktionsraum eingesetzt, ergibt sich die maximale Länge dieser Ausrüstungsteile durch den halben Durchmesser des kürzeren Reaktordurchmessers. Bevorzugt haben die Flügel, Blätter, Scheiben, Arme oder Anker mindestens einen Abstand von 0,01×D, besonders bevorzugt 0,1×D, wobei D die oben definierte Bedeutung hat, von der Wand des Reaktionsraumes.

Durch das zumindest eine bewegliche Mischorgan in dem Reaktionsraum wird die Vermischung der Edukte, von denen der eine Eduktstrom über die n ≥1 von parallel zur Achse des Rohrreaktors ausgerichteten Düsen und der zweite Eduktstrom über den verbleibenden freien Raum dem Reaktionsraum zugeführt wird, verbessert.

Im Fall der Anordnung des zumindest einen beweglichen Mischorgans auf der Achse der zumindest einen parallel zur Achse des Reaktionsraumes angebrachten Düse ist dies dadurch bedingt, dass der die parallel zur Achse des Rohrreaktors ausgerichtete Düse verlassende Eduktstrahl aufgeweitet wird und sich somit schneller mit dem den freien Raum verlassenden Eduktstrom vermischt.

Im Fall der Ausführungsform, bei der sich im Reaktor n > 1 parallel zur Achse des Reaktionsraumes ausgerichtete Düsen und m ≥1 bewegliche Mischorgane befinden (wobei n und m jeweils ganze positive Zahlen sind und bevorzugt symmetrisch in Bezug auf die Reaktorachse angeordnet sind), bewirken die m ≥1 beweglichen Mischorgane durch Turbulenzerhöhung und Verdrallung der Strömung eine Intensivierung der Vermischung der Eduktgasströme.

Somit ist es aufgrund des zumindest einen beweglichen Mischorgans in dem Reaktionsraum möglich, bei gleicher Eintrittsgeschwindigkeit der Reaktanden den Durchmesser der Düsen zu erhöhen, ohne dass dadurch eine Verringerung der Vermischungsgeschwindigkeit des Strahls mit den negativen Folgen der Verlängerung der Mischzeit und Verbreitung der Kontaktzeit erfolgt. Insbesondere überraschend ist, dass bereits ein langsam laufendes bewegliches Mischorgan eine ausreichende zusätzliche Turbulenz erzeugt, die zu einer Verkürzung der Mischstrecke um bis zu 40% führt.

Bei dem erfindungsgemäßen Verfahren können primäre Amine verwendet werden, die bevorzugt ohne Zersetzung in die Gasphase überführt werden können. Besonders geeignet sind Amine, insbesondere Diamine, auf Basis von aliphatischen oder cycloaliphatischen Kohlenwasserstoffen mit 1 bis 15 Kohlenstoffatomen. Besonders gut geeignete Amine sind 1,6-Diamino-hexan, 1-Amino-3,3,5-trimethyl-5-aminomethylcyclohexan (IPDA) und 4,4'-Diaminodicyclohexylamin. Bevorzugt verwendet wird 1,6-Diaminohexan (HDA).

Ebenfalls bevorzugt können für das erfindungsgemäße Verfahren aromatische Amine verwendet werden, die bevorzugt ohne Zersetzung in die Gasphase überführt werden können. Beispiele für bevorzugte aromatische Amine sind Toluylendiamin (TDA), insbesondere 2,4- TDA und 2,6-TDA und Gemische daraus, Diaminobenzol, Naphthyldiamin (NDA) und 2,2'-, 2,4'-oder 4,4'-Methylendiphenyldiamin (MDA) oder Isomerengemische davon. Besonders bevorzugt ist Toluylendiamin (TDA), insbesondere 2,4-TDA und 2,6-TDA und deren Gemische.

Die Ausgangsamine werden vor der Durchführung des erfindungsgemäßen Verfahrens in der Regel verdampft und auf 200°C bis 600°C, bevorzugt 200°C bis 500°C, besonders bevorzugt 250°C bis 450°C erhitzt und gegebenenfalls verdünnt mit einem Inertgas wie $N_2$, He, Ar oder mit den Dämpfen eines inerten Lösungsmittels, z.B. aromatischen Kohlenwasserstoffen ggf. mit Halogensubstitution, wie z.B. Chlorbenzol oder o-Dichlorbenzol, dem Reaktionsraum zugeführt.

Die Verdampfung der Ausgangsamine kann in allen bekannten Verdampfungsapparaturen erfolgen. Bevorzugt werden Verdampfungssysteme eingesetzt, bei denen ein kleiner Arbeitsinhalt mit einer hohen Umwälzleistung über einen Fallfilmverdampfer geführt wird und dabei zur Minimierung der thermischen Belastung der Ausgangsamine der Verdampfungsvorgang - wie oben ausgeführt- ggf. durch Zuspeisung von Inertgas und / oder Dämpfen eines inerten Lösungsmittels unterstützt wird.

In einer besonders bevorzugten Ausführungsform des erfmdungsgemäßen Verfahrens werden Verdampfungssysteme eingesetzt, bei denen ein kleiner Arbeitsinhalt über zumindest einen Mikrowärmeaustauscher oder Mikroverdampfer umgewälzt wird. Der Einsatz entsprechender Wärmeaustauscher für die Verdampfung von Aminen wird z.B. in EP-A-1 754 698 offenbart. Bevorzugt werden in dem erfindungsgemäßen Verfahren die in den Absätzen [0007] bis [0008] und [0017] bis [0039] der EP-A-1 754 698 offenbarten Apparate eingesetzt.

Die dampfförmigen Amine können noch Anteile an unverdampften Tröpfchen der Amine (Aerosole) enthalten. Bevorzugt enthalten die dampfförmigen Amine jedoch im wesentlichen keine Tröpfchen an unverdampften Aminen, das heißt maximal 0,5 Gew.-% des Amins, besonders bevorzugt maximal 0,05 Gew.-% des Amins, bezogen auf das Gesamtgewicht des Amins, liegt in der Form von unverdampften Tröpfchen vor und der restliche Teil des Amins liegt dampfförmig vor. Ganz besonders bevorzugt enthalten die dampfförmigen Amine keine Tröpfchen an unverdampften Aminen.

Weiterhin erfolgt die Verdampfung und Überhitzung der Ausgangsamine bevorzugt mehrstufig, um unverdampfte Tröpfchen im dampfförmigen Aminstrom zu vermeiden. Insbesondere bevorzugt sind mehrstufige Verdampfungs- und Überhitzungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind z.B. beschrieben in "Droplet Separation", A. Bürkholz, VCH Verlagsgesellschaft, Weinheim - New York - Basel - Cambridge, 1989. Nach Verlassen des in Strömungsrichtung letzten Überhitzers wird das auf seine Solltemperatur vorgeheizte dampfförmige Amin mit einer mittleren Verweilzeit von bevorzugt 0,01 bis 60s, ganz besonders bevorzugt von 0,01 bis 30s, insbesondere bevorzugt 0,01-15 s, dem Reaktor bzw. der Düsenanordnung zur Umsetzung zugeführt. Dabei wird über technische Maßnahmen, z.B. eine ausreichende Isolierung zur Vermeidung von Abstrahlungsverlusten, der Gefahr einer erneuten Tröpfchenbildung begegnet. Durch die Erzeugung eines im Wesentlichen tröpfchenfreien dampfförmigen Aminstroms vor Eintritt in den Reaktor wird die Reaktorlaufzeit deutlich erhöht.

Bei dem erfindungsgemäßen Verfahren ist es vorteilhaft, Phosgen im Überschuss bezüglich der umzusetzenden Amingruppen einzusetzen. Bevorzugt liegt ein molares Verhältnis von Phosgen zu Amingruppen von 1,1:1 bis 20:1, bevorzugt 1,2:1 bis 5:1 vor. Auch das Phosgen wird auf Temperaturen von 200°C bis 600°C erhitzt und gegebenenfalls verdünnt mit einem Inertgas wie $N_2$, He, Ar oder mit den Dämpfen eines inerten Lösungsmittels, z.B. aromatischen Kohlenwasserstoffen ohne oder mit Halogensubstitution, wie z.B. Chlorbenzol oder o-Dichlorbenzol, dem Reaktionsraum zugeführt.

Bei dem erfindungsgemäße Verfahren werden die getrennt aufgeheizten Reaktionspartner wie oben beschrieben über eine Düsenanordnung in den Reaktionsraum eines Rohrreaktors eingeführt und bevorzugt adiabatisch und unter Beachtung geeigneter Reaktionszeiten umgesetzt. Anschließend wird bevorzugt durch Abkühlung des Reaktionsgemisches das Isocyanat kondensiert, wobei die Abkühlung des Reaktionsgemisches bis zu einer Temperatur oberhalb der Zersetzungstemperatur des entsprechenden Carbaminsäurechlorids erfolgt.

Die notwendige Verweilzeit zur vollständigen Umsetzung der eingesetzten Amine mit dem Phosgen zu den entsprechenden Isocyanaten liegt üblicherweise zwischen 0,05 und 15 Sekunden, in Abhängigkeit von der Art des eingesetzten Amins, der Starttemperatur, der adiabaten Temperaturerhöhung im Reaktionsraum, dem molaren Verhältnis von eingesetztem Amin und Phosgen, einer etwaigen Verdünnung der Reaktionspartner mit Inertgasen sowie dem gewählten Reaktionsdruck.

Wird für das jeweilige System (eingesetztes Amin, Starttemperatur, adiabate Temperaturerhöhung, molares Verhältnis der Reaktanden, Verdünnungsgas, Reaktionsdruck) eine einmal ermittelte Mindestverweilzeit für die vollständige Reaktion um weniger als 20%, vorzugsweise weniger als 10% überschritten, kann - wie oben ausgeführt - die Bildung von Folge-Reaktionsprodukten wie Isocyanuraten und Carbodiimiden weitgehend vermieden werden.

Bevorzugt weisen weder der Reaktionsraum noch die Düsenanordnung Heizflächen auf, die Anlass zu einer thermischen Belastung mit der Folge von Folgereaktionen wie Isocyanurat- oder Carbodiimidbildung geben können, oder Kühlflächen auf, die Anlass zu einer Kondensation mit der Folge der Ausbildung von Ablagerungen geben können. Die Edukte Phosgen und Amin werden so, abgesehen von etwaigen Abstrahlungs- und Ableitungsverlusten, bevorzugt adiabat umgesetzt. Dabei wird die adiabate Temperaturerhöhung im Mischaggregat und Reaktor allein über die Temperaturen, Zusammensetzungen und relativen Dosierungen der Eduktströme sowie der Verweilzeit in den Mischaggregaten und den Reaktoren eingestellt.

In einer bevorzugten Ausführungsform des erfindungsgemäßen Verfahrens beträgt die Durchsatzkapazität des eingesetzten Reaktors bei den erfindungsgemäß geforderten Reaktionsbedingungen > 1 t Amin / h, bevorzugt 2 - 50 t Amin / h, besonders bevorzugt 2-12 t Amin / h. Besonders bevorzugt gelten diese Werte für Toluylendiamin. Unter Durchsatzkapazität ist dabei zu verstehen, dass in dem Reaktor die genannte Durchsatzkapazität an Amin pro h umgesetzt werden kann.

Nach der erfolgten Phosgenierungsreaktion im Reaktionsraum wird das gasförmige Reaktionsgemisch, das bevorzugt wenigstens ein Isocyanat, Phosgen und Chlorwasserstoff umfasst, bevorzugt von dem gebildeten Isocyanat befreit. Dies kann beispielsweise dadurch erfolgen, indem das den Reaktionsraum kontinuierlich verlassende Gemisch, bevorzugt wenigstens ein Isocyanat, Phosgen und Chlorwasserstoff umfassend, nach Verlassen des Reaktionsraumes einer Kondensation in einem inerten Lösungsmittel unterzogen wird, wie sie bereits für andere Gasphasenphosgenierungen empfohlen wurde (EP-A-0 749 958).

Bevorzugt erfolgt die Kondensation jedoch dadurch, dass der in dem erfindungsgemäßen Verfahren eingesetzte Reaktionsraum zumindest eine Zone aufweist, in die zum Abbruch der Umsetzung der Amine und des Phosgens zu den entsprechenden Isocyanaten ein oder mehrere geeignete Flüssigkeitsströme ("Quench-Flüssigkeiten") eingesprüht werden. Dadurch kann, wie in EP-A-1 403 248 beschrieben, eine rasche Abkühlung der Gasgemische ohne den Einsatz kalter Flächen durchgeführt werden.

In einer besonders bevorzugten Form des erfindungsgemäßen Verfahrens ist die zumindest eine Zone (Kühlzone) in eine Quenchstufe, wie sie z. B. in EP-A-1 403 248 offenbart wurde, integriert. In einer besonders bevorzugten Form kommen mehrere Kühlzonen zum Einsatz, erfolgen Integration und Betrieb dieser zumindest zwei Kühlzonen mit einer Quenchstufe, wie hinsichtlich Aufbau und Betrieb in EP-A-1 935 875 offenbart.

Statt des integrierten Verbundes von der zumindest einen Kühlzone eines Reaktors mit einer Quenchstufe, wie sie in der EP-A-1 935 875 offenbart wurde, ist ebenfalls der entsprechende integrierte Verbund der Kühlzonen mehrerer Reaktoren mit einer Quenchstufe möglich. Bevorzugt ist jedoch der integrierte Verbund eines Reaktors mit zumindest einer Kühlzone mit einer Quenchstufe.

Unabhängig von der Art der gewählten Abkühlung wird die Temperatur der zumindest einen Abkühlzone bevorzugt so gewählt, dass sie einerseits oberhalb der Zersetzungstemperatur des dem Isocyanat entsprechenden Carbamoylchlorids liegt. Andererseits sollen sich Isocyanat und gegebenenfalls das in dem Amindampfstrom und / oder Phosgenstrom als Verdünnungsmittel mit verwendete Lösungsmittel weitestgehend kondensieren bzw. sich weitestgehend in dem Lösungsmittel lösen, während überschüssiges Phosgen, Chlorwasserstoff und ggf. als Verdünnungsmittel mit verwendetes Inertgas die Kondensations- bzw. Quenchstufe weitestgehend nicht kondensiert bzw. gelöst durchlaufen. Zur selektiven Gewinnung des Isocyanats aus dem gasförmigen Reaktionsgemisch besonders gut geeignet sind bei einer Temperatur von 80 bis 200°C, vorzugsweise 80 bis 180 °C gehaltene Lösungsmittel wie beispielsweise Chlorbenzol und / oder Dichlorbenzol, oder in diesen Temperaturbereichen gehaltenes Isocyanat oder Mischungen des Isocyanats mit Chlorbenzol und / oder Dichlorbenzol. Es ist für den Fachmann aufgrund der physikalischen Daten bei gegebener Temperatur, Druck und Zusammensetzung einfach vorhersagbar, welcher Massenanteil des Isocyanates in der Quenche kondensiert bzw. diese nicht kondensiert durchläuft. Ebenso ist es einfach vorherzusagen, welcher Massenanteil des überschüssigen Phosgens, Chlorwasserstoffs und ggf. als Verdünnungsmittel verwendeten Inertgases die Quenche unkondensiert durchläuft bzw. sich in der Quencheflüssigkeit löst.

Die Erzeugung der für das erfindungsgemäße Verfahren bevorzugten Strömung des gasförmigen Reaktionsgemischs als Strömung ohne wesentliche Rückvermischung durch den Reaktionsraum wird durch ein Druckgefälle über den Reaktionsraum sichergestellt. Bevorzugt besteht das Druckgefälle zwischen den Eduktzuleitungen vor der Vermischung einerseits und dem Ausgang aus der Kondensations- bzw. Quenchstufe andererseits. Bevorzugt liegt der absolute Druck in den EduktZuleitungen vor der Vermischung bei 200 bis 3000 mbar und hinter der Kondensations- bzw. Quenchstufe bei 150 bis 2500 mbar. Wesentlich ist hierbei jedoch lediglich die Aufrechterhaltung eines Differenzdrucks von den Eduktzuleitungen über den Reaktionsraum bis hinter die Kondensations- bzw. Quenchstufe von bevorzugt mindestens 50 mbar zwecks Gewährleistung der genannten gerichteten Strömung und einer guten Vermischung der Edukte.

Das die Kondensations- bzw. Quenchstufe verlassende Gasgemisch wird bevorzugt in einer nachgeschalteten Gaswäsche mit einer geeigneten Waschflüssigkeit von restlichem Isocyanat befreit, bevorzugt anschließend in an sich bekannter Weise von überschüssigem Phosgen befreit. Dies kann mittels einer Kühlfalle, Absorption in einem inerten Lösungsmittel (z.B. Chlorbenzol oder Dichlorbenzol) oder durch Adsorption und Hydrolyse an Aktivkohle erfolgen. Das die Phosgenrückgewinnungsstufe durchlaufende Chlorwasserstoffgas kann in an sich bekannter Weise zur Rückgewinnung des zur Phosgensynthese erforderlichen Chlors recyclisiert werden. Die nach ihrem Einsatz zur Gaswäsche anfallende Waschflüssigkeit wird dann bevorzugt zumindest teilweise als Quench-Flüssigkeit zur Abkühlung des Gasgemisches in die entsprechende Zone des Reaktionraumes eingesetzt.

Die Reindarstellung der Isocyanate erfolgt bevorzugt anschließend durch destillative Aufarbeitung der Lösungen bzw. Gemische aus der Kondensations- bzw. Quenchstufe.

Beispiele:

**Beispiel 1: Coldflow-Modell ohne Rührer:**

Durch ein Plexiglasrohr mit 54 mm Innendurchmesser strömt Luft unter Umgebungsbedingungen mit einer Geschwindigkeit von 5,5 m/s. Das Rohr mündet in eine Düse mit einem Durchmesser von 40 mm, in der Düse beträgt die Luftgeschwindigkeit 10 m/s. Die Luft tritt als Freistrahl aus der Düse in einen offenen Halbraum aus. Zur Bestimmung des effektiven Strahlaufweitungswinkels wird der Luftströmung über einen Injektor ein Nebelaerosol zugegeben und mittels Videomesstechnik an einer Position 717 mm stromab der Düsenmündung ein Strahldurchmesser von 167 mm gemessen. Umgerechnet entspricht dies einem effektiven Aufweitungswinkel des Düsenstrahls von 10,1° (Gesamtwinkel). Der so bestimmte effektive Aufweitungswinkel wird als Maß für die Mischeffizienz der Düse herangezogen; bei Annahme einer gegebenen Außenströmung (Durchmesser des Ringraums um die Düse) erlaubt er die Berechnung des Mischungswegs von Strahl und Außenströmung.

**Beispiel 2: Coldflow-Modell mit Rührer:**

Durch das in Beispiel 1 eingesetzte Plexiglasrohr mit 54 mm Innendurchmesser strömt Luft unter Umgebungsbedingungen mit einer Geschwindigkeit von 5,5 m/s. Auf der Achse des Rohrs befindet sich eine drehbar gelagerte Welle, an deren der Strömung zugewandtem Ende ein Propeller mit sechs unter einem Winkel von 45° angestellten Blättern und einem Durchmesser von 50 mm befestigt ist. Stromabwärts des Propellers mündet das Rohr in eine Düse mit einem Durchmesser von 40 mm, die Luft tritt als Freistrahl aus der Düse in einen offenen Halbraum aus. Die Welle auf der Achse des Rohrs reicht bis zu einer Position 20 mm stromabwärts der Düsenmündung; auf ihr ist an dieser Stelle ein Rührer mit 6 parallel zur Strömungsrichtung des aus der Düsenmündung austretenden Gases ausgerichteten Blättern und einem Durchmesser von 40 mm montiert. Der am strömungszugewandten Ende der Welle befindliche Propeller wird durch die Strömung in Bewegung versetzt und gibt diese Bewegung über die Welle an den stromabwärts der Düse befindlichen Rührer weiter. Durch den zentrifugal fördernden Rührer wird der mit einer axialen Geschwindigkeit von 10 m/s aus der Düse austretenden Luft eine von der Rotationsachse nach außen gerichtete radiale Geschwindigkeitskomponente aufgeprägt. Zur Bestimmung des effektiven Strahlaufweitungswinkels wird der Luftströmung stromaufwärts des Antriebspropellers über einen Injektor ein Nebelaerosol zugegeben und mittels Videomesstechnik an einer Position 717 mm stromab der Düsenmündung ein Strahldurchmesser von 253 mm gemessen. Umgerechnet entspricht dies einem effektiven Aufweitungswinkel des Düsenstrahls von 16,9° (Gesamtwinkel). Dieser im Vergleich zu Beispiel 1 deutlich größere Aufweitungswinkel des Düsenstrahls führt bei Annahme einer gegebenen Außenströmung (Durchmesser des Ringraums um die Düse) zu einem entsprechend kürzeren Mischungsweg des Strahls mit dieser Außenströmung. Das Verhältnis der Mischungsweglängen lässt sich bei konstant angenommenem Durchmesser des Ringraums um die Düse gemäß der folgenden Formel berechnen:

$$\frac{\text{Mischungsweglänge Beispiel 2}}{\text{Mischungsweglänge Beispiel 1}} = \frac{\tan(0{,}5 \cdot \text{Aufweitungswinkel Beispiel 1})}{\tan(0{,}5 \cdot \text{Aufweitungswinkel Beispiel 2})}$$

In diesem Fall beträgt die Mischungsweglänge für Beispiel 2 demzufolge nur 60 % der Mischungsweglänge für Beispiel 1, der Rührer bewirkt also eine Verkürzung der Mischungsweglänge um 40 %.

**Beispiel 3: Phosgenierung von TDA (erfindungsgemäß):**

Über eine Düse werden als Eduktstrom A 1,9 t/h eines Gemisches aus dampfförmigen 2,4 - und 2,6-Toluylendiamin (80:20) und über den die Düse umgebenden freien Raum als Eduktstrom P Phosgen mit gasförmiger HCl einem rotationssymmetrischen Reaktionsraum zugeführt. Die Eduktströme A und P werden jeweils getrennt auf oberhalb 300°C erhitzt. In dem Reaktionsraum befindet sich stromab der Düse ein Rührer, der über eine gelagerte Welle an der Düse befestigt ist, wobei der Rührer durch den Impuls der aus der Düse austretenden Strömung angetrieben wird. Die Welle durchdringt die Reaktorwand nicht. Der Rührer enthält 6 Blätter, die gleichmäßig über den Kreis verteilt sind. Die Umsetzung in dem Reaktionsraum erfolgt adiabatisch innerhalb einer Verweilzeit von weniger als 10 Sekunden wobei sich eine Reaktoraustrittstemperatur von ca. 430°C einstellt. Das Gasgemisch wird durch eine Kondensationsstufe geleitet und dabei auf eine Gastemperatur von ca. 165 °C abgekühlt. Das anfallende Kondensat wird einer Destillationssequenz zugeführt und ergibt reines TDI. Das nicht kondensierte Gasgemisch wird in einer anschließenden Wäsche mit o-Dichlorbenzol gewaschen und das Nebenprodukt HCl vom überschüssigen Phosgen absorptiv getrennt. Das bei der Wäsche anfallende o-Dichlorbenzol wird in dem Kondensationsschritt eingesetzt.

Die Druckdifferenz zwischen dem Druck in der TDA-Zuleitung und dem Druck am Gasaustritt aus der Kondensationsstufe beträgt 10 mbar, um eine gerichtete Gasströmung von den Zuleitungen zu erreichen.

Nach 200 Stunden Versuchszeit beträgt der Differenzdruck 11 mbar und ist damit im Rahmen der Messgenauigkeit großtechnischer Messinstrumente unverändert. Eine Inspektion zeigte keine Ablagerungen von Feststoffen.

## Patentansprüche

1. Verfahren zur Herstellung primärer Isocyanate durch Umsetzung der entsprechenden primären Amine mit Phosgen, **dadurch gekennzeichnet, dass** die primären Amine oberhalb der Siedetemperatur der Amine mit Phosgen in einem Rohrreaktor, der einen Reaktionsraum enthält, umgesetzt werden, bei dem

   a) mindestens ein Phosgen enthaltender Eduktstrom P und mindestens ein die Amine enthaltender Eduktstrom A dem Reaktionsraum über eine Düsenanordnung zugeführt werden, wobei die Düsenanordnung eine Anzahl von $n \geq 1$ parallel zur Rotationsachse des Rohrreaktors ausgerichteten Düsen und einen die Düsen umgebenden freien Raum umfasst, und
   b) einer der Eduktströme A oder P über die Düsen und der andere Eduktstrom über den die Düsen umgebenden freien Raum dem Reaktionsraum zugeführt werden, und
   c) der Reaktionsraum zumindest ein bewegliches Mischorgan enthält.

2. Verfahren nach Anspruch 1, bei dem das zumindest eine bewegliche Mischorgan ein Rührer ist.

3. Verfahren nach Anspruch 1, bei dem das zumindest eine Mischorgan keine die Wand des Rohrreaktors durchdringenden beweglichen Ausrüstungteile aufweist.

4. Verfahren nach Anspruch 1, bei dem das zumindest eine Mischorgan keinen externen Antrieb hat.

5. Verfahren nach Anspruch 1, bei dem das zumindest eine Mischorgan durch den Impuls mindestens eines der Eduktströme A und / oder P angetrieben wird.

6. Verfahren nach Anspruch 1, bei dem das zumindest eine Mischorgan über eine Magnetkupplung mit einem externen Antrieb verbunden ist.

7. Verfahren nach Anspruch 1, bei dem der mindestens eine die Amine enthaltenden Eduktstrom A dem Reaktionsraum über die Düsen und der mindestens eine Phosgen enthaltende Eduktstrom P dem Reaktionsraum über den die Düsen umgebenden freien Raum zugeführt wird.

8. Verfahren nach Anspruch 1, bei dem die Umsetzung der Amine mit dem Phosgen unter adiabatischer Reaktionsführung erfolgt.

9. Verfahren nach Anspruch 1, bei dem der Reaktionsraum rotationssymmetrisch ist und über seine gesamte Länge oder auch nur abschnittsweise eine sich in Strömungsrichtung erweiternde, gleichbleibende und/oder abnehmende durchströmte Querschnittsfläche aufweist.

10. Verfahren nach Anspruch 1, bei dem als primäre Amine Diaminohexan, Isophorondiamin, 2,4- und / oder 2,6-Toluylendiamin, Methylendiphenyldiamin, Naphthyldiamin oder Gemische daraus eingesetzt werden.

11. Verfahren nach Anspruch 1, bei dem der Reaktor eine Durchsatzkapazität von > 1 t an Amin / h aufweist.

## Claims

1. Method for producing primary isocyanates by reacting the corresponding primary amines with phosgene, **characterized in that** the primary amines are reacted at above the boiling temperature of the amines with phosgene in a tubular reactor which contains a reaction chamber, in which method

a) at least one starting material stream P containing phosgene and at least one starting material stream A containing the amines are supplied to the reaction chamber via a nozzle arrangement, the nozzle arrangement comprising a number n ≥1 of nozzles oriented parallel to the axis of rotation of the tubular reactor and an open space surrounding the nozzles, and
b) one of starting material streams A or P is supplied to the reaction chamber via the nozzles and the other starting material stream via the open space surrounding the nozzles, and
c) the reaction chamber contains at least one mobile mixing element.

2. Method according to Claim 1, in which the at least one mobile mixing element is a stirrer.

3. Method according to Claim 1, in which the at least one mixing element does not comprise any mobile equipment parts which pass through the wall of the tubular reactor.

4. Method according to Claim 1, in which the at least one mixing element does not have an external drive.

5. Method according to Claim 1, in which the at least one mixing element is driven by the momentum of at least one of the starting material streams A and/or P.

6. Method according to Claim 1, in which the at least one mixing element is connected with an external drive via a magnetic coupling.

7. Method according to Claim 1, in which the at least one starting material stream A containing the amines is supplied to the reaction chamber via the nozzles and the at least one starting material stream P containing phosgene is supplied to the reaction chamber via the open space surrounding the nozzles.

8. Method according to Claim 1, in which the amines react with the phosgene under adiabatic reaction control.

9. Method according to Claim 1, in which the reaction chamber is rotationally symmetrical and comprises a flow cross-sectional area which widens, remains constant or narrows in the direction of flow over its entire length or indeed only in places.

10. Method according to Claim 1, in which the primary amines used are diaminohexane, isophorone diamine, 2,4- and/or 2,6-toluene diamine, methylene diphenyl diamine, naphthyl diamine or mixtures thereof.

11. Method according to Claim 1, in which the reactor has a throughput capacity of > 1 t of amine/h.

**Revendications**

1. Procédé pour la préparation d'isocyanates primaires par transformation des amines primaires correspondantes avec du phosgène, **caractérisé en ce que** les amines primaires sont transformées avec du phosgène au-dessus de la température d'ébullition des amines dans un réacteur tubulaire, qui contient une chambre de réaction, dans lequel

a) au moins un flux de produits de départ P contenant du phosgène et au moins un flux de produits de départ A contenant les amines sont alimentés dans la chambre de réaction via une disposition de pulvérisation, la disposition de pulvérisation comprenant un nombre n ≥ 1 de pulvérisateurs orientés parallèlement à l'axe de rotation du réacteur tubulaire et un espace libre entourant les pulvérisateurs, et
b) un des flux de produits de départ A ou P est alimenté via les pulvérisateurs et l'autre flux de produits de départ est alimenté via l'espace libre entourant les pulvérisateurs dans la chambre de réaction, et
c) la chambre de réaction contient au moins un organe de mélange mobile.

2. Procédé selon la revendication 1, dans lequel ledit au moins un organe de mélange mobile est un agitateur.

3. Procédé selon la revendication 1, dans lequel ledit au moins un organe de mélange ne présente pas de pièces d'équipement mobiles traversant la paroi du réacteur tubulaire.

**4.** Procédé selon la revendication 1, dans lequel ledit au moins un organe de mélange mobile ne présente pas de dispositif d'entraînement externe.

**5.** Procédé selon la revendication 1, dans lequel ledit au moins un organe de mélange est entraîné par l'impulsion d'au moins un des flux de produits de départ A et/ou P.

**6.** Procédé selon la revendication 1, dans lequel ledit au moins un organe de mélange est assemblé via un couplage magnétique avec un dispositif d'entraînement externe.

**7.** Procédé selon la revendication 1, dans lequel ledit au moins un flux de produits de départ A contenant les amines est alimenté dans la chambre de réaction via les pulvérisateurs et ledit au moins un flux de produits de départ P contenant le phosgène est alimenté dans la chambre de réaction via l'espace libre entourant les pulvérisateurs.

**8.** Procédé selon la revendication 1, dans lequel la transformation des amines avec le phosgène a lieu en conduisant la réaction de manière adiabatique.

**9.** Procédé selon la revendication 1, dans lequel l'espace de réaction présente une symétrie de rotation et présente, sur toute sa longueur ou également seulement par sections, une surface transversale d'écoulement qui s'élargit, reste identique et/ou diminue dans le sens d'écoulement.

**10.** Procédé selon la revendication 1, dans lequel on utilise, comme amines primaires, du diaminohexane, de l'isophoronediamine, de la 2,4-toluylènediamine et/ou de la 2,6-toluylènediamine, de la méthylènediphényldiamine, de la naphtyldiamine ou des mélanges de ceux-ci.

**11.** Procédé selon la revendication 1, dans lequel le réacteur présente une capacité de débit > 1 t d'amine/h.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 1165831 A
- EP 570799 A
- EP 289840 A
- EP 1526129 A
- EP 1555258 A
- EP 1362847 A
- EP 1449826 A
- WO 2008055898 A
- EP 1319655 A
- EP 0291819 A
- WO 2007028715 A1
- EP 1754698 A
- EP 0749958 A
- EP 1403248 A
- EP 1935875 A

**In der Beschreibung aufgeführte Nicht-Patentliteratur**

- *Chem.-Ing.-Techn.,* 1972, vol. 44, 1051 ff
- *Appl.Sci.Res.,* 1953, vol. A3, 279
- *Chemie-Ing.-Techn.,* 1972, vol. 44, 1055
- **A. Bürkholz.** Droplet Separation. VCH Verlagsgesellschaft, 1989